(19)

Europäisches Patentamt

European Patent Office

Office européen des brevets

(11) **EP 3 708 175 A1**

(12) **EUROPEAN PATENT APPLICATION**
published in accordance with Art. 153(4) EPC

(43) Date of publication:
**16.09.2020 Bulletin 2020/38**

(21) Application number: **18876190.2**

(22) Date of filing: **09.11.2018**

(51) Int Cl.:
*A61K 35/28* (2015.01)    *A61P 17/02* (2006.01)
*A61P 25/00* (2006.01)    *A61P 37/02* (2006.01)
*C12N 5/0775* (2010.01)    *C12Q 1/6806* (2018.01)
*G01N 33/15* (2006.01)    *G01N 33/50* (2006.01)
*G01N 33/68* (2006.01)

(86) International application number:
**PCT/JP2018/041678**

(87) International publication number:
**WO 2019/093481 (16.05.2019 Gazette 2019/20)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.11.2017   JP 2017216356**

(71) Applicants:
• **Sapporo Medical University
Sapporo-shi, Hokkaido 060-8556 (JP)**
• **Nipro Corporation
Osaka-shi, Osaka 531-8510 (JP)**

(72) Inventors:
• **HONMOU, Osamu
Sapporo-shi
Hokkaido 060-8556 (JP)**

• **YOSHIKAWA, Yoshihiro
Osaka-shi
Osaka 531-8510 (JP)**
• **TOMII, Ryo
Osaka-shi
Osaka 531-8510 (JP)**
• **YAO, Masafumi
Osaka-shi
Osaka 531-8510 (JP)**
• **NISHII, Yukari
Osaka-shi
Osaka 531-8510 (JP)**
• **WAGATSUMA, Yusuke
Osaka-shi
Osaka 531-8510 (JP)**

(74) Representative: **Hoffmann Eitle
Patent- und Rechtsanwälte PartmbB
Arabellastraße 30
81925 München (DE)**

(54) **MEDICINAL PRODUCT FOR TISSUE REGENERATION, AND PREPARATION METHOD THEREFOR**

(57)    The present invention relates to a cell-based medicine comprising mesenchymal stem cells, and a method for producing the same. More specifically, the present invention relates to:
a cell-based medicine containing mesenchymal stem cells, wherein a) the mesenchymal stem cells express CX3CL1 under stimulation with an inflammatory cytokine, and/or b) 90% or more of the mesenchymal stem cells express EGFR and/or ITGA4; and
a method for producing a cell-based medicine containing mesenchymal stem cells, the method comprising the steps of: a) adding an inflammatory cytokine to a culture containing mesenchymal stem cells, and confirming that the mesenchymal stem cells express CX3CL1; and/or b) confirming that 90% or more of the mesenchymal stem cells express EGFR and/or ITGA4.

FIG. 1

**Description**

Technical Field

Related Applications

The present description includes the contents as described in the description of Japanese Patent Application No. 2017-216356 (filed on November 9, 2017), which serves as the basis for the right of priority of the present application.

Technical Field

The present invention relates to a cell-based medicine containing mesenchymal stem cells, and a method for producing the same. More specifically, the present invention relates to a cell-based medicine containing mesenchymal stem cells which is excellent in accumulating at a site of injury, immunomodulatory effect and neuroprotective effect and is suitable for tissue regenerative medicine, and a method for producing the same.

Background Art

Mesenchymal stem cells (MSCs) are known to have a protective effect on the brain (parenchyma and blood vessels). It has been confirmed using an experimental infarction model that MSC administration after cerebral infarction reduces infarct volume and improves behavioral functions (Non Patent Literatures 1 to 3 and Patent Literature 1). Numerous treatments of cerebral infarction patients by intravenous administration of MSCs have also been performed, and improvements in motor function and site of injury have been reported (Non Patent Literature 4 and Patent Literature 2). In spinal cord injury victims, intravenous administration of MSCs was also found to restore function, promote axonal regeneration, and reduce sites of injury.

A number of action mechanisms have been speculated regarding the treatment mechanism of MSCs, and these are classified into three groups: neurotrophic/protective effect by neurotrophic factors, angiogenic effect (restoration of cerebral blood flow), and nerve regeneration. The neurotrophic/protective effect is expected to be exerted via humoral factors such as BDNF (Brain Derived Neurotrophic Factor) and GDNF (Glial Derived Neurotrophic Factor) which are neurotrophic factors.

As to the neuroprotective effect against spinal cord injury, many neurotrophic factors and growth factors such as BDNF, NT-3, NGF, PDGF, and GDNF have been reported to be involved (Non Patent Literature 5), and Honmou et al. have confirmed the neuroprotective effect of BDNF in vivo (Non Patent Literatures 3 and 6). In addition, intravenous administration of MSCs showed axonal regeneration/sprouting of pyramidal and extrapyramidal tracts and protection of corticospinal tract neurons in the cerebral cortex. However, these effects are known to be further enhanced when MSCs whose genes have been manipulated to forcibly express BDNF are intravenously administered (Non Patent Literature 7).

There are two possible mechanisms for angiogenesis, one is that MSCs accumulated in the lesion secrete angiogenic factors and the like and induce angiogenesis, and the other is that the administered MSCs differentiate into vascular endothelium to form new blood vessels. There are also two possible mechanisms for nerve regeneration, one is that MSCs accumulated in the lesion promote endogenous neurogenesis, and the other is that the administered MSCs differentiate into nerve cells and glial cells.

As to the immunomodulatory effect of MSCs, it has been reported that microglia are modulated by TSG-6, TGF-$\beta$1, and CX3CL1, which are secreted by MSCs, and the microglia change from cytotoxic M1 type, which secretes inflammatory cytokines such as TNF-$\alpha$, IL-1$\beta$, and IL-6, to M2 type, which has a cytoprotective effect. (Non Patent Literatures 8 to 11). In addition, it has been reported that inflammation in nerve cells and glial cells is suppressed by IL-4, IL-13, BDNF, IGF, and the like secreted by M2 microglia, and as a result, secondary neuropathy associated with necrosis and apoptosis is suppressed (Non Patent Literatures 8 to 11). Furthermore, it has been reported that transplanted MSCs increase the expression of IL-4 and IL-13 at the site of spinal cord injury, while reducing TNF-$\alpha$ and IL-6, thereby inducing a switch from M1 macrophages, which have an inflammatory effect, to M2 macrophages, which have an anti-inflammatory effect, and promoting axonal regeneration and functional recovery after spinal cord injury (Non Patent Literature 12).

Citation List

Patent Literature

Patent Literature 1: WO 2002/000849
Patent Literature 2: WO 2009/002503

Non Patent Literature

Non Patent Literature 1: Iihoshi S. et al., Brain Res. 2004;1007:1-9.
Non Patent Literature 2: Nomura T. et al., Neuroscience. 2005;136:161-169.
Non Patent Literature 3: Honma T. et al., Exp. Neurol. 2006;199:56-66.
Non Patent Literature 4: Honmou O. et al., Brain. 2011;134:1790-1807.
Non Patent Literature 5: Hervey et al., 2015, Brain Resarch 1619: 36-71.
Non Patent Literature 6: Osaka et al., 2010, Brain Research 1343: 226-235.
Non Patent Literature 7: Sasaki et al., 2009, Journal of Neuroscience 29(47): 14932-14941.
Non Patent Literature 8: Giunti et al., 2012, Stem Cells 30, 2044-53.
Non Patent Literature 9: Yoo et al., 2013, Neurobiology of Disease 58, 249-257.
Non Patent Literature 10: Liu et al., 2014, Journal of Neuroinflammation 11, 135.
Non Patent Literature 11: Noh et al., 2016, Stem Cells Translatoinal Medicine 5, 1-12.
Non Patent Literature 12: Nakajima et al., 2012, Journal of Neurotrauma 29, 1614-25.

Summary of Invention

Technical Problem

An object of the present invention is to provide a cell-based medicine containing MSCs which has an excellent therapeutic effect, such as accumulating at the site of injury, immunomodulatory effect (inflammation modulatory effect) and neuroprotective effect, and a method for producing the same.

Solution to Problem

The inventors have studied an indicator for evaluating the function of MSCs in order to solve the above problem. The inventors have found that MSCs prepared for a cell-based medicine express CX3CL1 by cytokine stimulation, EGFR and/or ITGA4 expression is 90% or more, and the immunomodulatory ability (inflammation modulatory ability) and the ability to accumulate at the site of injury of MSCs can be evaluated using these as an indicator.

The present invention has been completed based on these findings, and includes the following (1) to (11).

(1) A method for producing a cell-based medicine comprising mesenchymal stem cells, the method comprising the steps of:

a) adding an inflammatory cytokine to a culture comprising mesenchymal stem cells, and confirming that the mesenchymal stem cells express CX3CL1, and/or
b) confirming that 90% or more of the mesenchymal stem cells express EGFR and/or ITGA4.

(2) The method according to (1), further comprising a step of confirming the ability to secrete one or more selected from BDNF, VEGF, and HGF in a culture comprising mesenchymal stem cells; the method preferably comprising a step of confirming the ability to secrete BDNF and/or VEGF, and more preferably comprising a step of confirming the ability to secrete BDNF. The secretion of BDNF, VEGF, and HGF may be either secretion from unstimulated cells or secretion from cells after stimulation with an inflammatory cytokine.

(3) The method according to (1) or (2), wherein the inflammatory cytokine is one or more selected from the group consisting of TNF-$\alpha$, INF$\gamma$, IL-1, IL-6, IL-8, IL-12, and IL-18.

(4) The method according to (1) or (2), wherein the inflammatory cytokine includes TNF-$\alpha$, INF$\gamma$, and IL-6; the inflammatory cytokine being preferably a mixture of TNF-$\alpha$, INF$\gamma$, and IL-6.

(5) A cell-based medicine comprising mesenchymal stem cells, wherein:

a) the mesenchymal stem cells express CX3CL1 under stimulation with an inflammatory cytokine, and/or
b) 90% or more of the mesenchymal stem cells express EGFR and/or ITGA4.

(6) The cell-based medicine according to (5), wherein the mesenchymal stem cells have the ability to secrete one or more selected from BDNF, VEGF, and HGF. The mesenchymal stem cells preferably have the ability to secrete BDNF and/or VEGF, and more preferably have the ability to secrete BDNF.

(7) The cell-based medicine according to (5) or (6), wherein the inflammatory cytokine is one or more selected from the group consisting of TNF-α, INFγ, IL-1, IL-6, IL-8, IL-12, and IL-18.

(8) The cell-based medicine according to (5) or (6), wherein the inflammatory cytokine includes TNF-α, INFγ, and IL-6.

(9) The cell-based medicine according to (5) or (6), wherein the CX3CL1 expression level by stimulation with a mixture of TNF-α, INFγ, and IL-6 is greater than the sum of CX3CL1 expression levels by stimulation with each TNF-α, INFγ, and IL-6 alone.

(10) A method for evaluating the immunomodulatory ability of a cell-based medicine comprising mesenchymal stem cells, the method comprising a step of stimulating the mesenchymal stem cells with an inflammatory cytokine and determining the expression of CX3CL1. The "inflammatory cytokine" is preferably one or more selected from the group consisting of TNF-α, INFγ, IL-1, IL-6, IL-8, IL-12, and IL-18, more preferable to include TNF-α, INFγ, and IL-6, and further preferable to include a mixture of TNF-α, INFγ, and IL-6.

(11) A method for evaluating the ability of a cell-based medicine comprising mesenchymal stem cells to accumulate at a site of injury, the method comprising a step of evaluating whether the expression of EGFR and/or ITGA4 in the mesenchymal stem cells is 90% or more.

Advantageous Effects of Invention

According to the present invention, it is possible to easily evaluate the immunomodulatory effect (inflammation modulatory effect), accumulation at a site of injury, and neuroprotective effect of MSCs, and to provide a cell-based medicine containing highly functional MSCs.

Brief Description of Drawings

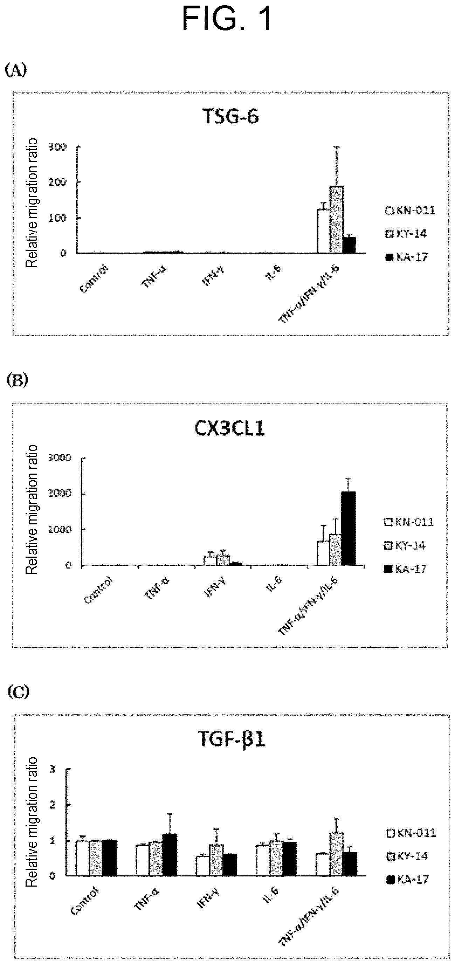

[Figure 1] Figure 1 shows the results of measuring by real-time RT-PCR the gene expression (relative expression ratio) of (A) TSG-6, (B) CX3CL1, and (C) TGF-β1 in MSC samples (KN-011, KY-14, KA-17, 3 Lots). The graphs show, from the left, no addition (Control), addition of TNF-α (50 ng/ml), IFN-γ (50 ng/ml), IL-6 (50 ng/mL) and TNF-a/IFN-γ/IL-6 (all 50 ng/ml).

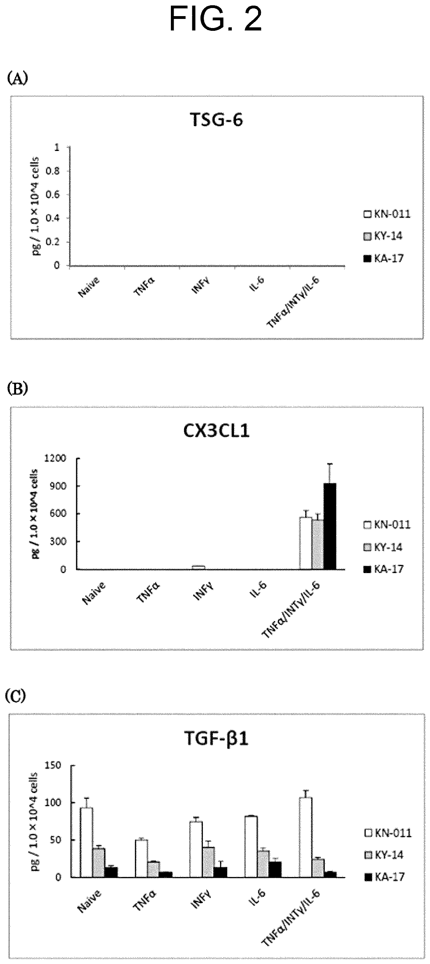

[Figure 2] Figure 2 shows the results of measuring by ELISA the expression levels (pg/1.0 x $10^4$ cells) of (A) TSG-6, (B) CX3CL1, and (C) TGF-β1 in MSC samples (KN-011, KY-14, KA-17, 3 Lots). The graphs show, from the left, no addition (Naive), addition of TNF-α (50 ng/ml), IFN-γ (50 ng/ml), IL-6 (50 ng/mL) and TNF-α/IFN-γ/IL-6 (all 50 ng/ml).

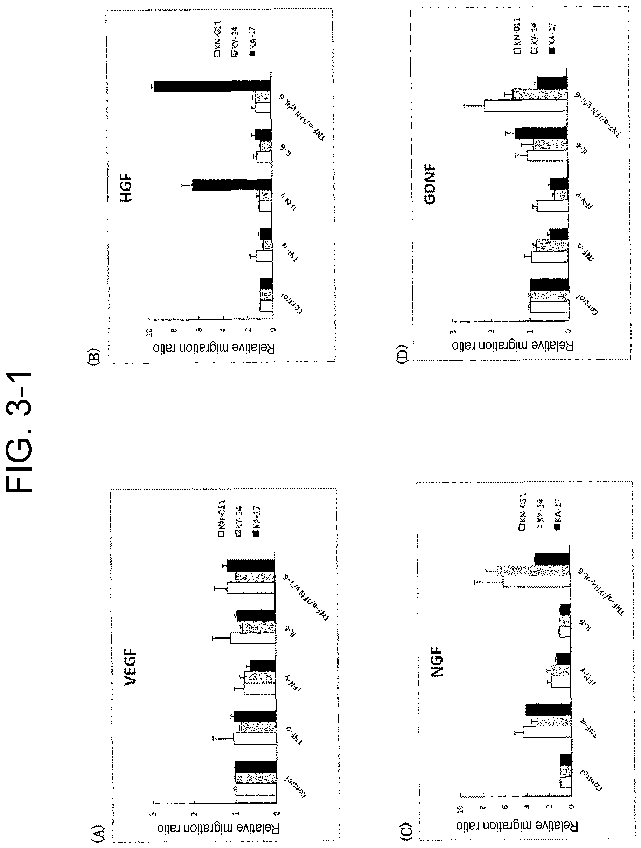

[Figure 3-1] Figure 3-1 shows the results of measuring by real-time RT-PCR the gene expression (relative expression ratio) of (A) VEGF, (B) HGF, (C) NGF, and (D) GDNF in MSC samples (KN-011, KY-14, KA-17, 3 Lots). The graphs show, from the left, no addition (Control), addition of TNF-α (50 ng/ml), IFN-γ (50 ng/ml), IL-6 (50 ng/mL) and TNF-α/IFN-γ/IL-6 (all 50 ng/ml).

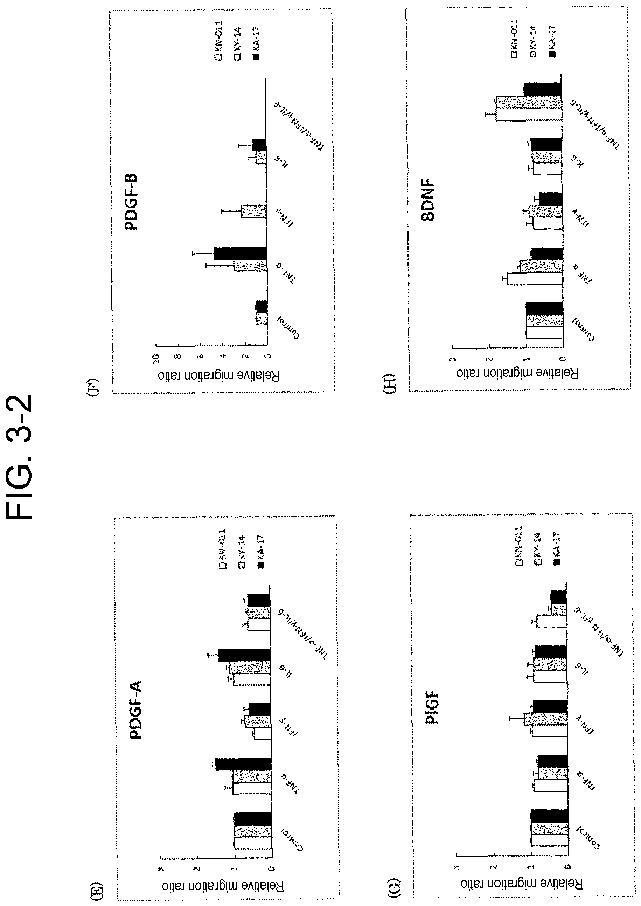

[Figure 3-2] Figure 3-2 shows the results of measuring by real-time RT-PCR the gene expression (relative expression ratio) of (E) PDGF-A, (F) PDGF-B, (G)PIGF, and (H) BDNF in MSC samples (KN-011, KY-14, KA-17, 3 Lots). The graphs show, from the left, no addition (Control), addition of TNF-α (50 ng/ml), IFN-γ (50 ng/ml), IL-6 (50 ng/mL) and TNF-α/IFN-γ/IL-6 (all 50 ng/ml).

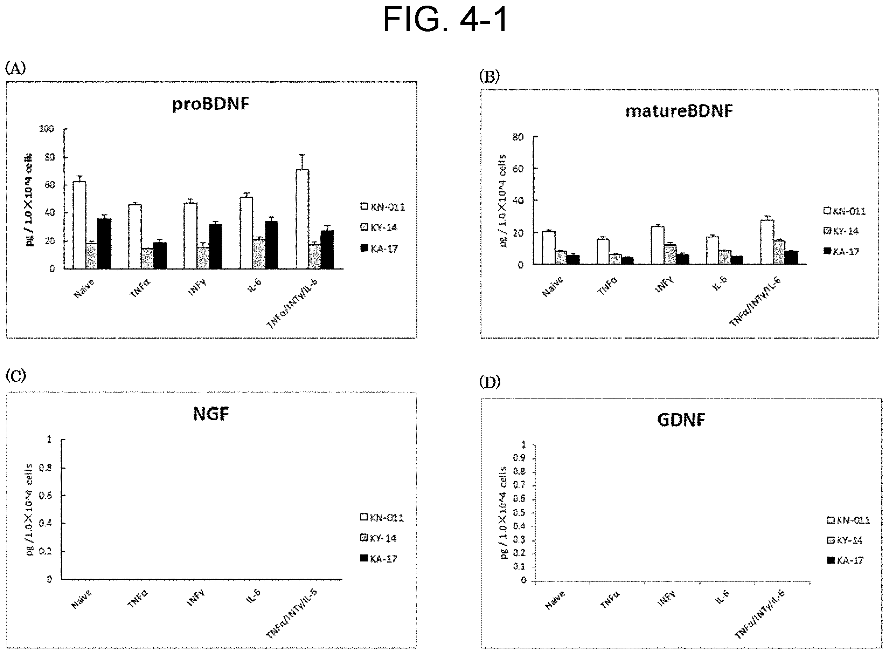

[Figure 4-1] Figure 4-1 shows the results of measuring by ELISA the expression levels (pg/1.0 x $10^4$ cells) of (A) proBDNF, (B) mature PDNF, (C) NGF, and (D) GDNF in MSC samples (KN-011, KY-14, KA-17, 3 Lots). The graphs show, from the left, no addition (Naive), addition of TNF-α (50 ng/ml), IFN-γ (50 ng/ml), IL-6 (50 ng/mL) and TNF-α/IFN-y/IL-6 (all 50 ng/ml).

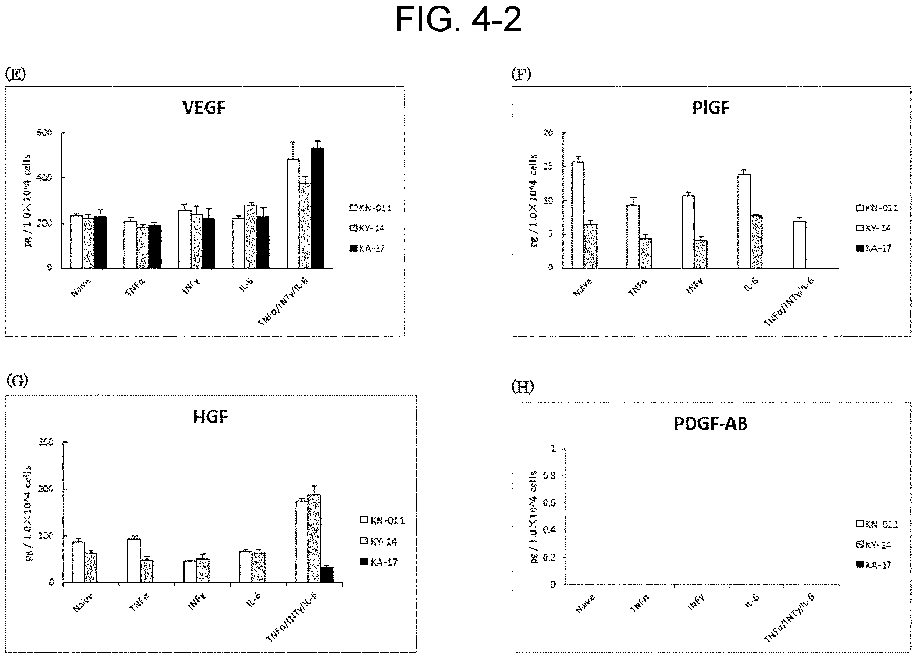

[Figure 4-2] Figure 4-2 shows the results of measuring by ELISA the expression levels (pg/1.0 x $10^4$ cells) of (E) VEGF, (F) PIGF, (G) HGF, and (H) PDGF-AB in MSC samples (KN-011, KY-14, KA-17, 3 Lots). The graphs show, from the left, no addition (Naive), addition of TNF-α (50 ng/ml), IFN-γ (50 ng/ml), IL-6 (50 ng/mL) and TNF-α/IFN-y/IL-6 (all 50 ng/ml).

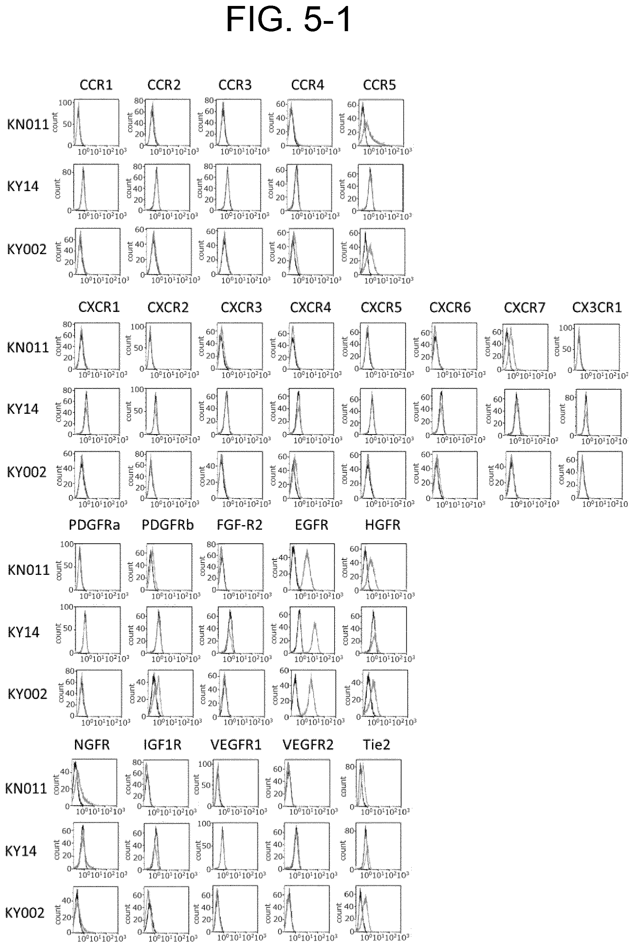

[Figure 5-1] Figure 5-1 shows the results of expression analysis by flow cytometry of chemokine receptors (CCR1, CCR2, CCR3, CCR4, CCR5, CXCR1, CXCR2, CXCR3, CXCR4, CXCR5, CXCR6, CXCR7, and CX3CR1) and growth factor receptors (PDGFRa, PDGFRb, FGF-R2, EGFR, HGFR, NGFR, IGF1R, VEGFR1, VEGFR2, and Tie-2) in MSC samples (KN-011, KY-14, KA-17, 3 Lots).

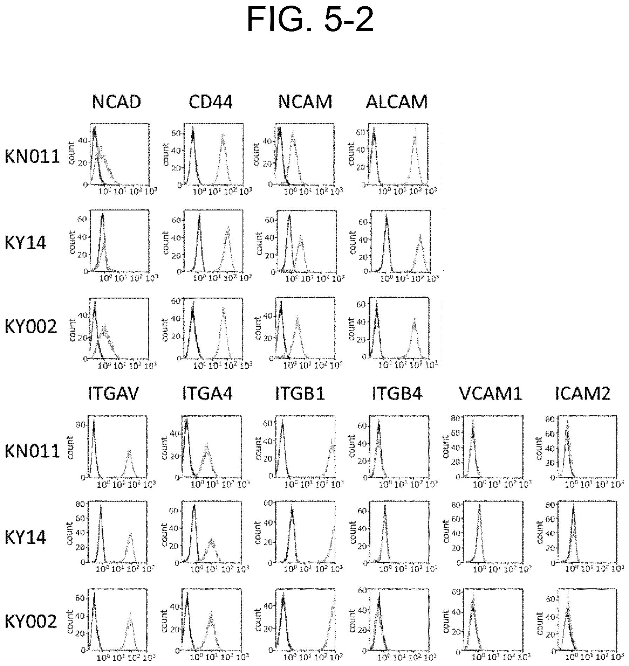

[Figure 5-2] Figure 5-2 shows the results of expression analysis by flow cytometry of adhesion factors (NCAD, HCAM (CD44), NCAM, ALCAM, ITGAV, ITGA4, ITGB1, ITGB4, VCAM1, and ICAM2) in MSC samples (KN-011, KY-14, KA-17, 3 Lots).

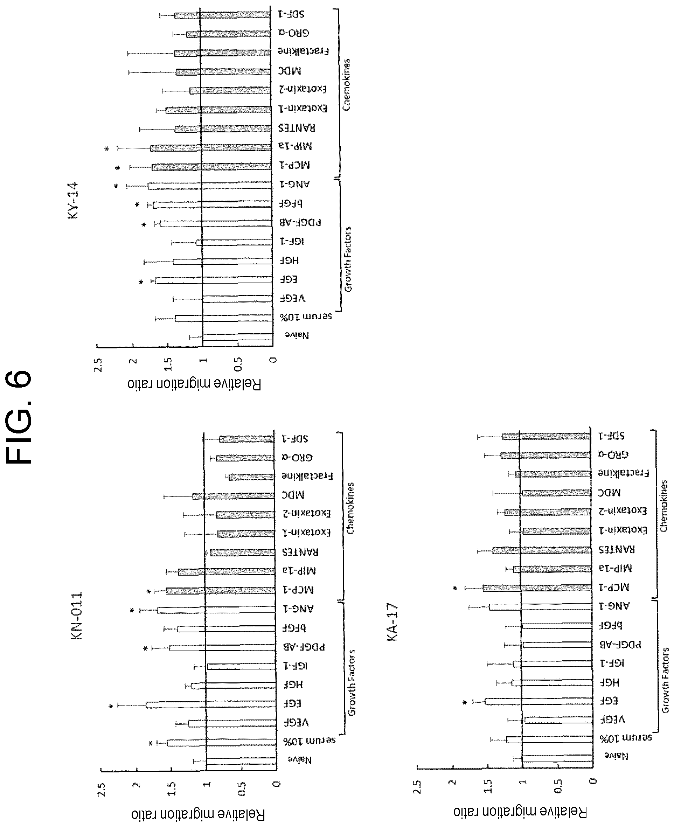

[Figure 6] Figure 6 shows the results of Migration Assay of MSC samples (KN-011, KY-14, KA-17, 3 Lots) by chemokine and growth factor stimulation (relative expression ratio to unstimulated culture, Mean $\pm$ SD, *1.5 fold-change v.s Naive).

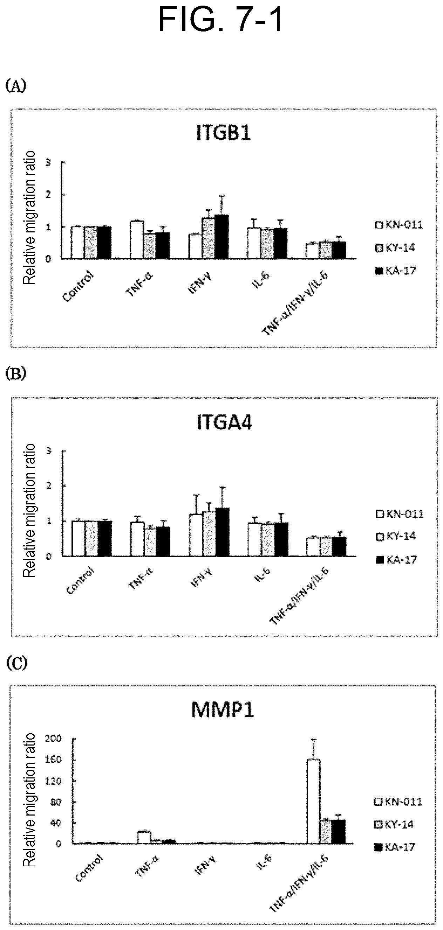

[Figure 7-1] Figure 7-1 shows the results of measuring by real-time RT-PCR the gene expression (relative expression ratio) of adhesion factors ((A) ITGB1 and (B) ITGA4), and infiltration-related proteins ((C) MMP1) in MSC samples (KN-011, KY-14, KA-17, 3 Lots). The graphs show, from the left, no addition (Control), addition of TNF-$\alpha$ (50 ng/ml), IFN-$\gamma$ (50 ng/ml), IL-6 (50 ng/mL) and TNF-$\alpha$/IFN-y/IL-6 (all 50 ng/ml).

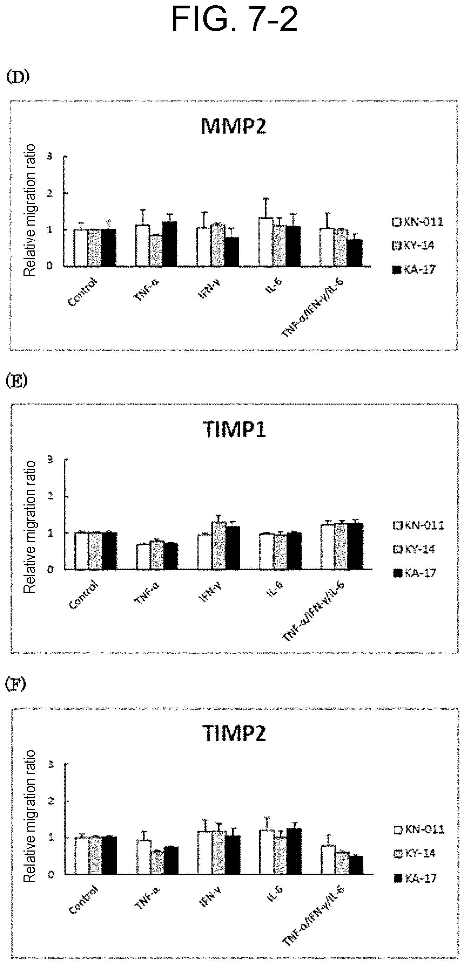

[Figure 7-2] Figure 7-2 shows the results of measuring by real-time RT-PCR the gene expression (relative expression ratio) of infiltration-related proteins ((D) MMP2, (E) TIMP1, and (F) TIMP2) in MSC samples (KN-011, KY-14, KA-17, 3 Lots). The graphs show, from the left, no addition (Control), addition of TNF-$\alpha$ (50 ng/ml), IFN-$\gamma$ (50 ng/ml), IL-6 (50 ng/mL) and TNF-$\alpha$/IFN-$\gamma$/IL-6 (all 50 ng/ml).

Description of Embodiments

1. Cell-based medicine containing mesenchymal stem cells

The cell-based medicine of the present invention contains mesenchymal stem cells, and is characterized in that: a) the mesenchymal stem cells express CX3CL1 under cytokine stimulation, and/or b) 90% or more of the mesenchymal stem cells express EGFR and/or ITGA4.

[Mesenchymal stem cells]

The "mesenchymal stem cells" used in the present invention are stem cells having pluripotency and self-renewal ability which are present in trace amounts among stromal cells of mesenchymal tissue, and are known to have the ability to differentiate not only into connective tissue cells such as osteocytes, chondrocytes, and lipocytes, but also into nerve cells and cardiomyocytes.

As to the therapeutic mechanism of MSCs, numerous action mechanisms have been speculated and proposed. For example, MSCs are known to enable effective tissue regeneration by accumulating at the site of injury. In addition, it has been reported that MSCs have an inhibitory effect on cell death and an inflammation modulatory effect, and have a neuroprotective effect via the secretion of neurotrophic factors (as mentioned above).

[CX3CL1 Expression]

The MSCs used in the present invention are characterized by expressing CX3CL1 upon stimulation of an inflammatory cytokine.

CX3CL1 is a chemokine of the CXXXC motif, also called fractalkine, expressed in activated vascular endothelial cells, nerve cells, dendritic cells and intestinal epithelial cells, and its expression is induced by stimulation with an inflammatory cytokine. Chemokines refer to a group of basic bioactive peptides having a molecular weight of about 10 kDa, which have chemotactic activity for leukocytes such as neutrophils, monocytes, and lymphocytes, and play an important role in inflammatory reactions. Chemokines are classified into four subfamilies, CXC, CC, C, and CX3C, based on their structural characteristics, and a seven-transmembrane trimeric G protein-coupled receptor (GPCR) family classified into CXCR, CCR, XCR, and CX3CR has been identified for these chemokine subfamilies. In vivo, CX3CL1 takes two forms, a membrane-bound form and a secreted form, and functions not only as a chemokine but also as a cell adhesion molecule showing integrin-independent cell adhesion ability. The expression of CX3CL1 is known to be involved in various diseases such as rheumatoid arthritis and arteriosclerosis.

The inhibitory effect on cell death and the inflammation modulatory effect are known to be related to the modulation effect of microglia and macrophage by MSCs at the site of injury, but the inventors have found that the characteristic expression of CX3CL1 in response to stimulation with an inflammatory cytokine is useful as an indicator of MSC's inflammation modulatory effect (immunomodulatory effect), by real-time RT-PCR and ELISA analysis. MSCs expressing CX3CL1 are expected to exert an immunomodulatory (inflammation modulatory) effect by modulating microglia/macrophages and inducing a switch from M1 type having an inflammatory effect to M2 type having an anti-inflammatory effect.

Examples of the "inflammatory cytokine" to be used include interleukins such as IL-1, IL-6, IL-8, IL-12, and IL-18, TNF-$\alpha$, and IFN-$\gamma$. Among these, IL-6, TNF-$\alpha$ and IFN-$\gamma$ are preferable, and it is more preferable to use a mixture of IL-6, TNF-$\alpha$ and IFN-$\gamma$.

If MSCs express CX3CL1 in response to stimulation with an inflammatory cytokine, the MSCs can be expected to have an excellent inflammation modulatory effect (immunomodulatory effect).

In particular, the fact that when stimulated using TNF-$\alpha$, INF$\gamma$, and IL-6, the CX3CL1 expression level by stimulation with a mixture of TNF-α, INFγ, and IL-6 is greater than the sum of CX3CL1 expression levels by stimulation with each TNF-α, INFγ, and IL-6 alone can be used as a characteristic indicator of functional (having an excellent inflammation modulatory effect) MSCs.

[EGFR and/or ITGA4 Expression]

The MSCs used in the present invention are characterized in that 90% or more express EGFR (Epidermal Growth Factor Receptor) and/or ITGA4 (Integrin subunit Alpha 4).

EGFR is a type of tyrosine kinase receptor and binds to TGF-α, amphiregulin, and the like in addition to epidermal growth factor (EGF) as a ligand. Receptor tyrosine kinases such as EGFR transmit stimulation with extracellular growth factors into cells and transmit the stimulation to the nucleus by signal transduction. As a result, the transcriptional activity in the nucleus is increased, which alters protein synthesis and the function and structure of cells. EGFR is known to play an important role in the proliferation of various cells and in the development and formation of organs in the body.

Integrins are one of the cell surface proteins and are mainly cell adhesion molecules involved in the cell adhesion to the extracellular matrix and signal transduction from the extracellular matrix. An integrin molecule is a heterodimer in which an α-chain and a β-chain are associated at a ratio of 1: 1. At least 18 types of α-chains have been reported, and ITGA4 is one of them. ITGB1 and ITGA4 are important for the adhesion to vascular endothelium and have been reported to be related to the accumulation of migrated cells at the site of injury (James et al., 2007, Brigitte et al., 2006).

Accumulation at the site of injury involves the migratory ability of MSCs. The present inventors performed receptor analysis by flow cytometry and migration assay on chemokines and growth factors related to migration, and found the expression of EGFR and/or ITGA4 to be useful as an indicator of the migration ability and ability to accumulate at the site of injury of MSCs.

The secretion of growth factors and the like such as EGF and NGF is known to increase at the site of tissue injury in trauma patients. In addition, the release of EGF, bFGF, IL-6 and IL-8 from blood platelets during the wound healing process has been confirmed (Ono et al., 1995, Burns 21, 352-355, Zhuang et al., 2013, Asian Pacific Journal of Tropical Medicine, 383-386, Werner et al., 2003, Physiol Rev 83, 835-870). However, there are no reports on the relationship between the expression of EGFR and ITGA4 in MSCs and the accumulation at the site of injury.

If the expression of EGFR and/or ITGA4 of MSCs is 90% or more, the MSCs can be expected to have an excellent ability to accumulate at the site of injury.

[BDNF Expression]

It is preferable that the MSCs used in the present invention secrete one or more trophic factors selected from BDNF, VEGF and HGF, in addition to the expression of CX3CL1, and the expression of EGFR and/or ITGA4. Among these, the secretion of BDNF and/or VEGF is important, and the secretion of BDNF is particularly important.

BDNF (Brain-derived Neurotrophic Factor) is a humoral protein that binds to a specific receptor TrkB on the surface of target cells and regulates the growth of nerve cells. BDNF acts on some neurons of the central nervous system and peripheral nervous system, promoting their maintenance and growth, and promoting differentiation into new neurons and synapses. In the brain, BDNF is activated in the hippocampus, cerebral cortex, and cerebral basal ganglia, and is important for long-term memory, but is also known to act on the retina, motor neurons, kidneys, salivary glands, and prostate.

VEGF (Vascular Endothelial Growth Factor) is a growth factor that specifically acts on vascular endothelial cells isolated from the culture of pituitary cells. Since VEGF has the effect of promoting angiogenic processes, including the proliferation of vascular endothelial cells, and of enhancing vascular permeability, it has been presumed to be related to various diseases and symptoms in which angiogenesis plays an important role (cancer, diabetic retinopathy, rheumatoid arthritis, wound healing process)

HGF (Hepatocyte Growth Factor) is a cytokine purified as a factor that strongly promotes the proliferation of primary cultured hepatocytes, and is an important factor that promotes liver regeneration. HGF exerts biological activity via c-Met receptors expressed on target cells, and promotes cell proliferation, cell motility, anti-apoptosis (cell death), morphogenesis induction and angiogenesis, not only for hepatocytes but also for various cells.

Trophic factors and the like secreted by MSCs may be involved in the neuroprotective effect. The present inventors have examined the expression of neurotrophic factors secreted by MSCs by real-time RP-PCR and ELISA, and have confirmed that the expression of BDNF, VEGF, and HGF, especially the expression of BDNF and/or VEGF, in particular the expression of BDNF, was useful as an indicator of the neuroprotective effect of MSCs.

If the MSCs have the ability to secrete BDNF, VEGF and/or HGF, it can be expected that the MSCs have the ability to repair and regenerate the injured area and have an excellent neuroprotective effect. Although MSCs secrete BDNF, VEGF and/or HGF even when not stimulated, the secretion ability may be confirmed by evaluating the secretion from unstimulated cells or by evaluating the secretion from cells after stimulation with an inflammatory cytokine.

As shown in the examples below, the MSCs used in the present invention also express TGF-β1 in addition to CX3CL1 as a factor involved in the inflammation modulatory effect (immunomodulatory effect). In addition, as factors involved in the migration ability, the expression of NCAM, ALCAM, ITGAV, and ITGB1 was also observed in addition to EGFR and/or ITGA4.

[Expression Analysis]

In the present invention, the expression of the above CX3CL1, EGFR, ITGA4, BDNF, VEGF, and HGF can be easily determined by a method well-known in the art. For example, real-time PCR (real-time RT-PCR), microarray, Northern blot, and the like can be utilized for expression analysis at the gene level. Moreover, ELISA, flow cytometry (FCM), protein chips, and the like can be utilized for expression analysis at the protein level.
In particular, for the expression at the protein level, in the case of cell surface proteins such as EGFR and ITGA4, it is preferable to use flow cytometry (FCM) in terms of simplicity and sensitivity, and in the case of secretory proteins such as CX3CL1, BDNF, VEGF, and HGF, it is preferable to use a bead-based assay in terms of simplicity and sensitivity.

[MSC Modulation]

The sources of the MSCs used in the present invention include bone marrow, peripheral blood, umbilical cord blood, fetal embryo, and the brain, but in the present invention, MSCs derived from human bone marrow or blood (bone marrow mesenchymal stem cells), particularly human bone marrow MSCs are preferable.
The cells may be cells induced to differentiate from ES cells or induced pluripotent stem cells (such as iPS cells), may be established cells, or may be cells isolated and proliferated from living organisms. The cells may be derived from allogeneic cells or derived from autologous cells, but autologous cell-derived (derived from the patient's own cells) MSCs are preferable.
In the MSCs used in the present invention, it is preferable that the expression of at least one or more selected from CD73, CD90, and CD105 is 90% or more, and/or the expression of CD34 or CD45 is 5% or less. More preferably, the MSCs used in the present invention are characterized in that the expression of at least two or more selected from CD73, CD90, and CD105 is 90% or more, and/or the expression of CD34 and CD45 is 5% or less. Further preferably, the MSCs used in the present invention are characterized in that the expression of CD73, CD90, and CD105 is 90% or more, and the expression of CD34 or CD45 is 5% or less.
Moreover, it is preferable that the MSCs used in the present invention are cells that are CD24 negative, which is a differentiation marker, and maintain an undifferentiated state. MSCs maintained in an undifferentiated state have the characteristic that the proliferation rate and the survival rate after introduction into a living body are high. A method for obtaining such undifferentiated MSCs has also been developed, and details thereof are described in WO 2009/034708.
The functional MSCs suitable for the cell-based medicine of the present invention can be prepared, for example, by proliferating cells separated from bone marrow fluid or the like under conditions such that they do not substantially come into contact with an anticoagulant (such as heparin), using a culture medium containing human serum (preferably autologous serum), and containing no anticoagulant or an extremely low concentration of an anticoagulant (such as heparin). Here, "containing no anticoagulant or an extremely low concentration of an anticoagulant" means that it does not contain an effective amount of an anticoagulant as an anticoagulant. Specifically, for example, in the case of heparin or a derivative thereof, the effective amount as an anticoagulant is usually about 20 to 40 U/mL, but in the above method, by minimizing the amount added to a blood collection tube for sampling in advance, the amount in a sample collected from a living body is less than 5 U/mL, preferably less than 2 U/mL, further preferably less than 0.2 U/mL, and the amount present in the culture medium when cells are cultured is less than 0.5 U/mL, preferably less than 0.2 U/mL, further preferably less than 0.02 U/mL, based on the volume of the culture medium (see WO 2009/034708).
The density of the cells in the culture medium affects the properties of the cells and the direction of differentiation. In the case of MSCs, if the cell density in the culture medium exceeds 8,500 cells/cm$^2$, the properties of the cells will change. Therefore, it is preferable to subculture at a maximum of 8,500 cells/cm$^2$ or less, and more preferably, to subculture when the cell density reaches 5,500 cells/cm$^2$ or more.
In the above method, since a culture medium containing human serum is used, it is desirable that the number of medium changes is as small as possible, taking into consideration the burden on the serum donor, and for example, the medium is changed at least once a week, more preferably once or twice a week.
As for the culture, subculture is repeated until the total number of cells reaches 10$^8$ cells or more. The number of cells required may vary depending on the purpose of use, but for example, the number of MSCs required for transplantation for the treatment of cerebral infarction is considered to be 10$^7$ cells or more. According to the above method, 10$^7$ MSCs can be obtained in about 12 days.
The proliferated MSCs may be stored by a technique such as cryopreservation (for example, in a deep freezer at -152°C) until use, if necessary. For cryopreservation, a culture medium (a culture medium for mammalian cells such as RPMI) containing serum (preferably human serum, more preferably autologous serum), dextran, and DMSO is used as a cryopreservation solution. For example, cells can be suspended in a cryopreservation solution containing 20.5 mL of RPMI sterilized by standard filtration, 20.5 mL of autologous serum collected from a patient, 5 mL of dextran, and 5 mL of DMSO, and cryopreserved at -150°C. For example, as DMSO, Cryoserv manufactured by Nipro Corporation can be used, and as dextran, low molecular weight dextran L injection manufactured by Otsuka Pharmaceutical can be used, but they are not limited thereto.

[Cell-based Medicine (Cell-based Preparation)]

The larger the number of MSCs contained in the cell-based medicine of the present invention is, the more preferable. However, when taking into account the time of administration to a subject and the time required for culturing, it is practical to use the minimum amount showing the effects. Therefore, in a preferable aspect of the present invention, the number of MSCs is $10^7$ or more, preferably $5 \times 10^7$ or more, more preferably $10^8$ or more, and further preferably $5 \times 10^8$ or more. The number of administrations is not limited to one, and may be two or more.

The cell-based medicine of the present invention is preferably a preparation for parenteral administration, more preferably a preparation for parenteral systemic administration, in particular a preparation for intravenous administration. Dosage forms suitable for parenteral administration include injections such as solution injections, suspension injections, emulsion injections, and extemporaneously prepared injections, and implants. Preparations for parenteral administration are in the form of an aqueous or non-aqueous isotonic sterile solution or suspension. For example, pharmacologically acceptable carriers or media, specifically, sterile water or normal saline solution, a culture medium (in particular, a culture medium used for culturing mammalian cells such as RPMI), physiological buffers such as PBS, vegetable oils, emulsifiers, suspending agents, surfactants, stabilizers, excipients, vehicles, preservatives, binding agents and the like are appropriately combined and formulated into an appropriate unit dosage form.

Examples of aqueous solutions for injection include normal saline solution, culture media, physiological buffers such as PBS, isotonic solutions containing glucose or other adjuvants, such as D-sorbitol, D-mannose, D-mannitol, and sodium chloride, and these may be used with a suitable solubilizing agent such as alcohol, specifically ethanol, polyalcohol, propylene glycol, polyethylene glycol, or a nonionic surfactant such as polysorbate 80 or HCO-50.

The cell-based medicine of the present invention is a medicine for tissue regeneration, and in particular, it is useful for treating dementia, chronic cerebral infarction, chronic spinal cord injury, neurodegenerative diseases, mental illnesses, higher dysfunctions and the like, by synapse formation and a plasticity promoting effect at the site of injury (lesion).

2. Method for producing a cell-based medicine containing mesenchymal stem cells

The present invention also provides a method for producing a cell-based medicine containing mesenchymal stem cells. The method for producing the cell-based medicine of the present invention includes the steps of: a) adding cytokines to a culture containing mesenchymal stem cells, and confirming that the mesenchymal stem cells express CX3CL1, and/or b) confirming that the mesenchymal stem cells express EGFR and/or ITGA4.

Examples of the "inflammatory cytokine" to be used include TNF-$\alpha$, INF$\gamma$, IL-1, IL-6, IL-8, IL-12, and IL-18, among these it is preferable to include TNF-$\alpha$, INF$\gamma$, and IL-6, and more preferable to use a mixture of TNF-$\alpha$, INF$\gamma$, and IL-6.

The method for producing the cell-based medicine of the present invention may further include a step of confirming the presence of one or more selected from BDNF, VEGF, and HGF in the culture (without cytokines). In particular, it is important to confirm the presence of BDNF and/or VEGF, and it is most important to confirm the presence of BDNF.

As described above, if MSCs express CX3CL1 by the addition of inflammatory cytokines, the MSCs can be expected to have an excellent inflammation modulatory effect (immunomodulatory effect), and if 90% or more of the MSCs express EGFR and/or ITGA4, the MSCs can be expected to have an excellent ability to accumulate at the site of injury. In addition, if any of the trophic factors such as BDNF, VEGF, and HGF is present in the culture medium, MSCs having a high neuroprotective effect can be expected to be contained, and among these, the presence of BDNF and/or VEGF, especially the presence of BDNF may be an important indicator of MSCs having a high neuroprotective effect. Although MSCs secrete BDNF, VEGF and/or HGF even when not stimulated, the secretion ability may be confirmed by evaluating the secretion from unstimulated cells or by evaluating the secretion from cells after stimulation with an inflammatory cytokine.

It is preferable to use the expression at the protein level rather than the gene level as an indicator for the expression of the above CX3CL1, EGFR, ITGA4, BDNF, VEGF, and HGF, which can be determined by the method described in the previous section. In particular, in the case of cell surface proteins such as EGFR and ITGA4, it is preferable to use flow cytometry (FCM) in terms of simplicity and sensitivity, and in the case of secretory proteins such as CX3CL1, BDNF, VEGF, and HGF, it is preferable to use a bead-based assay in terms of simplicity and sensitivity.

The MSCs used in the method for producing the cell-based medicine of the present invention can be prepared by proliferating cells separated from bone marrow fluid or the like under conditions such that they do not substantially come into contact with an anticoagulant (such as heparin), using a culture medium containing human serum (preferably autologous serum), and containing no anticoagulant or an extremely low concentration of an anticoagulant (such as heparin), as described in the previous section, according to the description in WO 2009/034708. Here, "containing no anticoagulant or an extremely low concentration of an anticoagulant" means that it does not contain an effective amount of an anticoagulant as an anticoagulant. Specifically, for example, in the case of heparin or a derivative thereof, the effective amount as an anticoagulant is usually about 20 to 40 U/mL. In the above-described method, by minimizing the amount added to a blood collection tube for sampling in advance, the amount in a sample collected from a living body is less than 5 U/mL, preferably less than 2 U/mL, further preferably less than 0.2 U/mL, and the amount present in the medium when cells are cultured is less than 0.5 U/mL, preferably less than 0.2 U/mL, further preferably less than 0.02 U/mL, based on the volume of the culture medium.

3. Method for evaluating the immunomodulatory ability of a cell-based medicine containing mesenchymal stem cells

The present invention also provides a method for evaluating the immunomodulatory ability of a cell-based medicine containing mesenchymal stem cells. The evaluation method includes a step of stimulating mesenchymal stem cells with an inflammatory cytokine and determining the expression of CX3CL1. The "inflammatory cytokines" to be used and the method for determining the expression of CX3CL1 are as described in 1 and 2.

If the MSCs after cytokine stimulation express CX3CL1, the cell-based medicine containing the MSCs can be evaluated as having high immunomodulatory ability. In particular, if when stimulated using TNF-$\alpha$, INF$\gamma$, and IL-6, the CX3CL1 expression level by stimulation with a mixture of TNF-$\alpha$, INF$\gamma$, and IL-6 is greater than the sum of CX3CL1 expression levels by stimulation with each TNF-$\alpha$, INF$\gamma$, and IL-6 alone, the MSCs can be evaluated as having high immunomodulatory ability.

4. Method for evaluating the accumulation of a cell-based medicine containing mesenchymal stem cells at a site of injury

A method for evaluating the ability of a cell-based medicine containing mesenchymal stem cells to accumulate at a site of injury is also provided. The evaluation method includes a step of confirming that 90% or more of the mesenchymal stem cells express EGFR and/or ITGA4.

If 90% or more of the MSCs express EGFR and/or ITGA4, a cell-based medicine containing the MSCs can be evaluated as having an excellent ability to accumulate at the site of injury.

Examples

Hereafter, the present invention is described specifically with examples, but the present invention is not limited to these examples.

Example 1: Immunomodulatory effect

The inhibitory effect on cell death and immunomodulatory effect of MSCs is known to be related to the modulation effect of microglia and macrophages by MSCs at the site of injury. Therefore, in order to examine the immunomodulatory ability of a cell-based medicine containing MSCs, the expression of TSG-6, CX3CL1, and TGF-$\beta$1 as relevant factors was analyzed by real-time RT-PCR and ELISA.

1. Experimental methods and evaluation items 1.1 Cell culture

As MSC samples, samples of three different lots for clinical trial (STRO1) (KN-011, KY-14, and KA-17) were used. The MSC samples were suspended in 14 mL of a culture solution (10% human serum, 1% Penicillin-streptomysin, 1% L-Glutamine) and seeded on a 150 mm dish at a density of 0.7 to 1.0 x $10^6$ cells/dish. The cells were cultured under the conditions of a temperature of 37°C and 5% $CO_2$, and after confirming about 80% confluency, the cells were subcultured and seeded at a density of 5.0 x $10^5$ cells/dish. Subculture was continued, and the cells were seeded at a density of 3.0 x $10^5$ cells/dish on a 100 mm dish at the fourth passage. Four passages of cells were used in all of the following experimental systems.

1.2 Collection of culture supernatant stimulated with inflammatory cytokines and extraction of total RNA

Twenty-four hours after the fourth passage, the culture solution was replaced with a normal culture solution (10% FBS, 1% Penicillin-Streptomycin, 1% L-Glutamine), and 10 mL of a culture solution with inflammatory cytokines (TNF-$\alpha$ (50 ng/ml), IFN-$\gamma$ (50 ng/ml), IL-6 (50 ng/ml), and TNF-$\alpha$/IFN-$\gamma$/IL-6 (50 ng/ml each) (5 Conditions, n = 3). The inflammatory cytokines are thought to be secreted at the site of spinal cord injury and to cause various cell disorders. Forty-eight hours after the exchange, the culture supernatant was collected and centrifuged (2280 g, 20 min). Thereafter, 200 $\mu$l of each was dispensed into 1.5 ml tubes and stored in a -80°C freezer. After collecting the supernatant, the cells were detached from the dish by trypsin treatment, and were counted. After counting the cells, total RNA was extracted using an RNA extraction kit (QIAGEN). cDNA was synthesized from total RNA, and real-time RT-PCR was performed using these as templates.

2. Evaluation of results and criteria

2.1 Gene expression by real-time RT-PCR

cDNA was synthesized from total RNA extracted from cells. A PCR reaction was performed with the synthesized cDNA as templates, using Taqman probes for each of the factors TSG-6, CX3CL1, and TGF-$\beta$1. Based on the Ct value of each target and the internal standard, the gene expression levels of cells cultured with a normal culture solution and with inflammatory cytokines were compared and quantified by the $\Delta\Delta$Ct method.

The test specimens were a specimen free of cytokines (Naive), and specimens with TNF-$\alpha$ (50 ng/ml), IFN-$\gamma$ (50 ng/ml), IL-6 (50 ng/ml), and TNF-$\alpha$/IFN-$\gamma$/IL-6 (50 ng/ml each). mRNA and culture supernatant were collected 48 hours after the start of culture, and real-time RT-PCR was performed.

2.2 Quantitation of secreted proteins by ELISA

Using the culture supernatant collected and stored in 1.1 as a sample, the TSG-6, CX3CL1, and TGF-$\beta$1 in the culture supernatant were quantified. The test specimens were a specimen free of cytokines (Naive), and specimens with TNF-$\alpha$ (50 ng/ml), IFN-$\gamma$ (50 ng/ml), IL-6 (50 ng/ml), and TNF-$\alpha$/IFN-$\gamma$/IL-6 (50 ng/ml each). mRNA and culture supernatant were collected 48 hours after the start of culture, and real-time RT-PCR was performed.

3. Results

3.1 Real-time RT-PCR (Figure 1)

The graph shows the relative expression ratio to the control, and the table shows the Ct value. Gene expression of TSG-6, CX3CL1, and TGF-$\beta$1 was confirmed in all lots, and in particular, the expression of TSG-6 and CX3CL1 was significantly increased by the addition of the mixture of TNF-$\alpha$/IFN-$\gamma$/IL-6. These results suggest that MSCs are involved in the modulation effect on microglia and macrophages, and that TSG-6, CX3CL1, and TGF-$\beta$1 contribute to this effect.

3.2 Quantitation of secretory proteins by ELISA (Figure 2)

In all lots, TSG-6 and TGF-$\beta$1 showed no change in the expression level due to cytokine stimulation. However, although CX3CL1 showed almost no expression when unstimulated or stimulated with a cytokine alone, a significant expression was observed by the addition of the mixture of TNF-$\alpha$/IFN-$\gamma$/IL-6. In addition, in the RT-PCR results, the expression by the mixed stimulation with TNF-$\alpha$/IFN-$\gamma$/IL-6 was greater than the sum of the expressions by each stimulation alone.

4. Discussion

The expression of CX3CL1 was hardly observed by ELISA, but the expression by the mixed stimulation with TNF-$\alpha$/IFN-$\gamma$/IL-6 was confirmed. In addition, the expression by mixed stimulation with TNF-$\alpha$/IFN-$\gamma$/IL-6 is greater than the sum of expressions by each stimulation alone; this expression characteristic of CX3CL1 was not observed for other immunomodulatory ability-related factors secreted by MSCs (TSG-6 and TGF-$\beta$1). Therefore, CX3CL1 was considered to be useful as an indicator for evaluating the immunomodulatory ability of MSCs.

Example 2: Neuroprotective effect

A plurality of trophic factors may be involved in the neuroprotective effect of MSCs. The expression of trophic factors (VEGF, HGF, NGF, GDNF, PDGF-A, PDGF-A, PIGF, and BDNF) secreted by MSCs was analyzed.

1. Experimental methods and evaluation items

The cell culture and preparation of total RNA were performed by the method described in Example 1.

2. Evaluation of results and criteria

Same as in Example 1.

3. Results

3.1 Real-time RT-PCR (Figure 3)

The graph shows the relative expression ratio to the control, and the table shows the Ct value. In MSCs cultured in a culture solution free of inflammatory cytokines, the expression of BDNF, NGF, and GDNF, which are neurotrophic factors, VEGF, PDGF-A, and PIGF, which are involved in angiogenesis, and HGF, which is involved in the repair and regeneration of damaged tissues, was confirmed. With the mixed stimulation with TNF-$\alpha$/IFN-y/IL-6, the expression of NGF was found to have a tendency to increase.

3.2 Quantitation of secretory proteins by ELISA (Figure 4)

In MSCs cultured in a culture solution free of inflammatory cytokines, the secretion of mature-BDNF, which is a neurotrophic factor, its precursor proBDNF, and VEGF, which is involved in angiogenesis, was confirmed. In addition, HGF and PIGF were confirmed in two out of three specimens. On the other hand, the secretion of NGF, GDNF and PDGF-AB was not confirmed. With the mixed stimulation with TNF-$\alpha$/IFN-$\gamma$/IL-6, the secretion amount of VEGF and HGF were found to have a tendency to increase.

4. Discussion

At the mRNA level, the expression of all trophic factors was confirmed, but at the protein level, secretion could be confirmed in all samples only for BDNF and VEGF. With PIGF and HGF, confirmation was possible in only two out of three lots.
As to the neuroprotective effect against spinal cord injury, numerous neurotrophic factors and growth factors such as BDNF, NT-3, NGF, PDGF, and GDNF have been reported to be involved, and the in vivo analysis of Honmou et al. has confirmed the neuroprotective effect of BDNF (cited previously, Nomura et al., 2005; Osaka et al., 2010). In addition, these effects are known to be further enhanced when intravenously administering BDNF-MSCs which have been genetically modified to forcibly express BDNF (cited previously, Sasaki et al., 2009,).
These results are consistent with the above report, and it is considered that BDNF secretion is particularly important as an evaluation indicator of the neuroprotective effect of MSCs. VEGF and HGF are also considered useful for the functional evaluation of MSCs in addition to BDNF.

Example 3: MSC migration ability

In order to evaluate the accumulation of MSCs at the site of injury, the in vitro migration ability of MSCs was analyzed by FCM method, Migration Assay and real-time RT-PCR method.

1. Experimental methods and evaluation items

The cell culture and preparation of total RNA were performed by the method described in Example 1.

2. Evaluation of results and criteria

2.1 Flow cytometry (FCM) method

First, as an analysis of chemokines and growth factors related to migration, the expression of each of the following receptors was analyzed using the FCM method.

<Receptors involved in migration>

Chemokine receptors:
CCR1, CCR2, CCR3, CCR4, CCR5, CXCR1, CXCR2, CXCR3, CXCR4, CXCR5, CXCR6, CXCR7, CX3CR1
Growth factor receptors:
VEGFR1, VEGFR2, PDGFR$\beta$, EGFR, IGF-1R, FGF-R2, HGFR, Tie-2

<Adhesion factors>

ICAM2, VCAM, ALCAM, HCAM (CD44), ITGAV, ITGA4, ITGB1

2.2 Migration Assay

Next, the chemokines and growth factors shown below were added to the culture medium, and the migration ability of MSCs was studied using the Migration Assay method.

<Chemokines and growth factors>

VEGF, EGF, HGF, IGF-1, PDGF-AB, bFGF, ANGPT-1
MCP-1 (CCL2), MIP-1$\alpha$ (CCL3), RANTES (CCL5), Eotaxin-1 (CCL11), MDC (CCL22),
Eotaxin-2 (CCL24), CRO-$\alpha$ (CXCL1), SDF-1 (CXCL12), Fractalkine (CX3CL1)
The Migration Assay was performed using FluoroBlok (Corning).

1) The migration factor was added to the well plate, and the insert was set,
2) the cell suspension was added to the top of the insert,
3) after 18 hours, the number of migrated cells was counted by counting the stained cells using Calcein AM (Dojindo Laboratories).

The results were evaluated by the relative migration ratio where the number of migrated cells when no chemokine or growth factor was added was 1.0.

```
Relative migration ratio = number of cells (with

migration factor)/number of cells (without migration

factor)
```

2.3 Real-time RT-PCR

According to Example 1, with respect to the factors relating to adhesion of cells to vascular endothelium and infiltration into tissues, gene expression with and without stimulation with an inflammatory cytokine was analyzed by real-time RT-PCR.

<Adhesion factors>

ITGB1, ITGA4

<Infiltration-related proteins>

MMP1, MMP2, TIMP1, TIMP2

3. Results

3.1 Analysis of chemokine receptors, growth factor receptors and adhesion factors of MSCs by FCM method (Table 1 and Figure 5)

For the chemokine receptors, the expression of CCR5, CXCR3, and the like was observed in some cells, but none was expressed in all cells. On the other hand, for the growth factor receptors, the expression of EGFR, HGFR, NGFR, and Tie2 was observed. For the adhesion factors, the expression of NCAD, CD44, NCAM, ALCAM, ITGA4, and ITGB1, which are considered to be involved in the adhesion of the migrated MSCs to vascular endothelial cells, was observed.

[Table 1]

Expression analysis of chemokine receptors and growth factor receptors by FCM method

| Cell | Chemokine receptor | | | | | | | | | | | | |
|---|---|---|---|---|---|---|---|---|---|---|---|---|---|
| | CCR1 | CCR2 | CCR3 | CCR4 | CCR5 | CXCR1 | CXCR2 | CXCR3 | CXCR4 | CXCR5 | CXCR6 | CXCR7 | CX3CR1 |
| KN011 | — | — | — | — | + | — | — | +/— | — | — | — | + | — |
| KY14 | — | — | — | — | — | — | — | — | — | — | — | — | — |
| KY002 | — | — | — | +/— | + | — | — | +/— | + | — | +/— | — | — |

| Cell | Growth factor receptor | | | | | | | | | |
|---|---|---|---|---|---|---|---|---|---|---|
| | PDGFRa | PDGFRb | FGF-R2 | EGFR | HGFR | NGFR | IGF1R | VEGFR1 | VEGFR2 | Tie2 |
| KN011 | — | +/— | — | + | + | + | — | — | — | +/— |
| KY14 | — | — | — | + | +/— | +/— | — | — | + | +/— |
| KY002 | — | — | — | + | + | +/— | — | — | — | + |

| Cell | Adhesion factor | | | | | | | | | |
|---|---|---|---|---|---|---|---|---|---|---|
| | NCAD | CD44 | NCAM | ALCAM | ITGAV | ITGA4 | ITGB1 | ITGB4 | VCAM1 | ICAM2 |
| KN011 | + | + | + | + | + | + | + | — | — | — |
| KY14 | +/— | + | + | + | + | + | + | — | — | — |
| KY002 | + | + | + | + | + | + | + | — | — | — |

— : Not expressed at all

+/— : Slightly expressed

+ : Expressed

3.2 Migration Assay (Figure 6)

EGF, PDGF-AB, βFGF, ANGPT-1, MCP-1 (CCL2), and MIP-1α (CCL3) were found to promote migration, and this tendency was especially significant with EGF and MCP-1 (CCL2).

3.3 Real-time RT-PCR (Figure 7)

It was confirmed that ITGB1 and ITGA4, which are adhesion factors, and MMP1, MMP2, TIMP1, and TIMP2, which are related to infiltration, were expressed. In addition, it was confirmed that MSCs stimulated with inflammatory cytokines (TNF-α/IFN-γ/IL-6) greatly increased the expression of MMP1. ITGB1 and ITGA4 are important for the adhesion to vascular endothelium and have been reported to be related to the accumulation of migrated cells at the site of injury (cited previously, James et al., 2007). In addition, it is known that the MMP and TIMP families thaw the basement membrane of cells, and migrated cells infiltrate the site of injury (Caroline et al., 2008, Mariusz et al., 2012). These reports and the above results suggested that the MSCs have properties related to the adhesion to vascular endothelium and infiltration into tissues.

4. Discussion

The results of the receptor analysis by FCM and of the Migration Assay confirmed that the expression of EGFR was particularly important as an indicator of MSC migration ability. Moreover, the results of the FCM analysis and real-time RT-PCR analysis confirmed that the expression of ITGA4 was important as an indicator of MSC migration ability.

Industrial Applicability

According to the present invention, the function of a cell-based medicine containing mesenchymal stem cells can be appropriately assayed, and a cell-based medicine containing mesenchymal stem cells suitable for tissue regeneration can be provided.

All publications, patents and patent applications cited in the present specification are hereby incorporated by reference in their entirety.

**Claims**

1. A method for producing a cell-based medicine comprising mesenchymal stem cells, the method comprising the steps of:

   a) adding an inflammatory cytokine to a culture comprising mesenchymal stem cells, and confirming that the mesenchymal stem cells express CX3CL1; and/or
   b) confirming that 90% or more of the mesenchymal stem cells express EGFR and/or ITGA4.

2. The method according to claim 1, further comprising a step of confirming the ability to secrete one or more selected from BDNF, VEGF, and HGF in a culture comprising mesenchymal stem cells.

3. The method according to claim 1 or 2, wherein the inflammatory cytokine is one or more selected from the group consisting of TNF-$\alpha$, INF$\gamma$, IL-1, IL-6, IL-8, IL-12, and IL-18.

4. The method according to claim 1 or 2, wherein the inflammatory cytokine includes TNF-$\alpha$, INF$\gamma$, and IL-6.

5. A cell-based medicine comprising mesenchymal stem cells, wherein

   a) the mesenchymal stem cells express CX3CL1 under stimulation with an inflammatory cytokine, and/or
   b) 90% or more of the mesenchymal stem cells express EGFR and/or ITGA4.

6. The cell-based medicine according to claim 5,
   wherein the mesenchymal stem cells have the ability to secrete one or more selected from BDNF, VEGF, and HGF.

7. The cell-based medicine according to claim 5 or 6, wherein the inflammatory cytokine is one or more selected from the group consisting of TNF-$\alpha$, INF$\gamma$, IL-1, IL-6, IL-8, IL-12, and IL-18.

8. The cell-based medicine according to claim 5 or 6, wherein the inflammatory cytokine includes TNF-$\alpha$, INF$\gamma$, and IL-6.

9. The cell-based medicine according to claim 5 or 6, wherein the CX3CL1 expression level by stimulation with a mixture of TNF-$\alpha$, INF$\gamma$, and IL-6 is greater than the sum of CX3CL1 expression levels by stimulation with each TNF-$\alpha$, INF$\gamma$, and IL-6 alone.

10. A method for evaluating the immunomodulatory ability of a cell-based medicine comprising mesenchymal stem cells, the method comprising a step of stimulating the mesenchymal stem cells with an inflammatory cytokine and determining the expression of CX3CL1.

11. A method for evaluating the ability of a cell-based medicine comprising mesenchymal stem cells to accumulate at a site of injury, the method comprising a step of evaluating whether the expression of EGFR and/or ITGA4 in the mesenchymal stem cells is 90% or more.

# FIG. 1

(A)

(B)

(C)

# FIG. 2

(A)

(B)

(C)

# FIG. 3-1

(A)

VEGF

(B)

HGF

(C)

NGF

(D)

GDNF

EP 3 708 175 A1

# FIG. 3-2

(E)

(F)

(G)

(H)

EP 3 708 175 A1

# FIG. 4-1

FIG. 4-2

(E)

VEGF

(F)

PIGF

(G)

HGF

(H)

PDGF-AB

# FIG. 5-1

# FIG. 5-2

FIG. 6

# FIG. 7-1

(A)

(B)

(C)

# FIG. 7-2

(D)

(E)

(F)

## INTERNATIONAL SEARCH REPORT

International application No.

PCT/JP2018/041678

### A. CLASSIFICATION OF SUBJECT MATTER

Int.Cl. A61K35/28(2015.01)i, A61P17/02(2006.01)i, A61P25/00(2006.01)i, A61P37/02(2006.01)i, C12N5/0775(2010.01)i, C12Q1/6806(2018.01)i, G01N33/15(2006.01)i, G01N33/50(2006.01)i, G01N33/68(2006.01)i

According to International Patent Classification (IPC) or to both national classification and IPC

### B. FIELDS SEARCHED

Minimum documentation searched (classification system followed by classification symbols)

Int.Cl. A61K35/28, A61P17/02, A61P25/00, A61P37/02, C12N5/0775, C12Q1/6806, G01N33/15, G01N33/50, G01N33/68

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

JSTPlus/JMEDPlus/JST7580 (JDreamIII), CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | WO 2017/170925 A1 (ROHTO PHARMACEUTICAL CO., LTD.) 05 October 2017, claim 6, examples, paragraph [0116], fig. 1 (Family: none) | 1, 3–9 |
| X | JP 2011-525798 A (UNIVERSITÀ DEGLI STUDI DI UDINE) 29 September 2011, claims 1, 25, examples 1A, 1B, 2A, 2B, paragraph [0054] & WO 2009/156495 A1, claims 1, 25, examples 1A, 1B, 2A, 2B, page 15, line 29 to page 16, line 2 & US 2011/0158962 A1 & EP 2313493 A1 & CN 102131918 A | 1, 3–9 |

☒ Further documents are listed in the continuation of Box C.   ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 February 2019 (01.02.2019) | 12 February 2019 (12.02.2019) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

## INTERNATIONAL SEARCH REPORT

International application No.

PCT/JP2018/041678

C (Continuation).   DOCUMENTS CONSIDERED TO BE RELEVANT

| Category* | Citation of document, with indication, where appropriate, of the relevant passages | Relevant to claim No. |
|---|---|---|
| Y<br>A | DU, W. J. et al., "Heterogeneity of proangiogenic features in mesenchymal stem cells derived from bone marrow, adipose tissue, umbilical cord, and placenta", Stem Cell Research & Therapy, 2016, 7:163, 11 pages, page 6, right column, lines 2-14, fig. 4 | 2-4, 6-9<br>6-9 |
| Y<br>A | MEAD, B. et al., "Paracrine-mediated neuroprotection and neuritogenesis of axotomised retinal ganglion cells by human dental pulp stem cells: comparison with human bone marrow and adipose-derived mesenchymal stem cells", PLOS ONE, 2014, vol. 9, no. 10, e109305 (11 pages), page 6, right column, lines 9-20, table 2, fig. 4 | 2-4, 6-9<br>6-9 |
| A | JP 2009-506769 A (AGENCY FOR SCIENCE, TECHNOLOGY AND RESEARCH) 19 February 2009, paragraph [0287], table 1  & WO 2007/027156 A1, page 55, table E1A & US 2008/0199849 A1 & EP 1937801 A1 & KR 10-2008-0056181 A & CN 101341244 A | 1, 5-9 |
| X<br>Y | WO 2009/034708 A1 (SAPPORO MEDICAL UNIVERSITY) 19 March 2009, claims 1-38, examples 1-19, paragraph [0122] & US 2010/0254953 A1, claims 1-5, 7-12, 16-19, 21-23, 36, 38, examples 1-19, paragraph [0198] & JP 2009-65854 A & EP 2194121 A1 & CN 101802174 A & KR 10-2016-0005140 A | 5-9<br>1-11 |
| Y | GIUNII, D. et al., "Mesenchymal stem cells shape microglia effector functions through the release of CX3CL1", Stem Cells, 2012, vol. 30, no. 9, pp. 2044-2053, abstract, page 2045, left column, "methods", paragraph [0001], page 2049, right column, lines 2-7 | 1-3, 5-10 |
| Y | CROITORU-LAMOURY, J. et al., "Human Mesenchymal Stem Cells Constitutively Express Chemokines and Chemokine Receptors That Can Be Upregulated by Cytokines, IFN-β, and Copaxone", Journal of Interferon & Cytokine Research, 2007, vol. 27, no. 1, pp. 53-64, abstract, fig. 2B | 1-3, 5-10 |
| Y | WO 2005/094888 A1 (TWO CELLS CO., LTD.) 13 October 2005, examples 1-3 & US 2008/0254019 A1, examples 1-3 & EP 1736173 A1 | 1-9, 11 |

Form PCT/ISA/210 (continuation of second sheet) (January 2015)

**INTERNATIONAL SEARCH REPORT**

| International application No. |
| --- |
| PCT/JP2018/041678 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2017-520537 A (CEDARS-SINAI MEDICAL CENTER) 27 July 2017, examples 2, 25 & WO 2015/187994 A1, examples 2, 25 & US 2017/0119823 A1 & EP 3151847 A1 | 1-9, 11 |
| A | KUMAR, S. et al., "Bone homing of mesenchymal stem cells by ectopic α4 integrin expression", FASEB Journal, 2007, vol. 21, no. 14, pp. 3917-3927, abstract, page 3920, right column, lines 27-29 | 1-9, 11 |
| A | CUI, L. L. et al., "Integrin α4 Overexpression on Rat Mesenchymal Stem Cells Enhances Transmigration and Reduces Cerebral Embolism After Intracarotid Injection", Stroke, October 2017, vol. 48, no. 10, pp. 2895-2900, summary, page 2895, right column, lines 2-4 | 1-9, 11 |
| P, A | WO 2018/159432 A1 (ROHTO PHARMACEUTICAL CO., LTD.) 07 September 2018, claims 1-6 (Family: none) | 1-9, 11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017216356 A
- WO 2002000849 A
- WO 2009002503 A
- WO 2009034708 A

### Non-patent literature cited in the description

- **IIHOSHI S. et al.** *Brain Res.,* 2004, vol. 1007, 1-9
- **NOMURA T. et al.** *Neuroscience,* 2005, vol. 136, 161-169
- **HONMA T. et al.** *Exp. Neurol.,* 2006, vol. 199, 56-66
- **HONMOU O. et al.** *Brain,* 2011, vol. 134, 1790-1807
- **HERVEY et al.** *Brain Resarch,* 2015, vol. 1619, 36-71
- **OSAKA et al.** *Brain Research,* 2010, vol. 1343, 226-235
- **SASAKI et al.** *Journal of Neuroscience,* 2009, vol. 29 (47), 14932-14941
- **GIUNTI et al.** *Stem Cells,* 2012, vol. 30, 2044-53
- **YOO et al.** *Neurobiology of Disease,* 2013, vol. 58, 249-257
- **LIU et al.** *Journal of Neuroinflammation,* 2014, vol. 11, 135
- **NOH et al.** *Stem Cells Translatoinal Medicine,* 2016, vol. 5, 1-12
- **NAKAJIMA et al.** *Journal of Neurotrauma,* 2012, vol. 29, 1614-25
- **ONO et al.** *Burns,* 1995, vol. 21, 352-355
- **ZHUANG et al.** *Asian Pacific Journal of Tropical Medicine,* 2013, 383-386
- **WERNER et al.** *Physiol Rev,* 2003, vol. 83, 835-870